# FASCICULE DE BREVET EUROPEEN

(11) **EP 3 148 747 B1**
(45) Date de publication et mention de la délivrance du brevet: **09.09.2020**
(21) Numéro de dépôt: 15732323.9
(22) Date de dépôt: 28.05.2015
(51) Int. Cl.: B25B 15/00, A61B 17/86, A61B 17/88, F16B 23/00

(54) **ORGANE DE SERRAGE A LOBES CONIQUES POUR VIS HEXALOBULAIRE ET VIS**
ANZIEHVORRICHTUNG MIT KONISCHEN LAPPEN FÜR HEXALOBULÄRE SCHRAUBE UND SCHRAUBE
TIGHTENING DEVICE WITH TAPERED LOBES FOR A HEXALOBULAR SCREW AND SCREW

(30) Priorité: 02.06.2014 FR 1454970
(43) Date de publication de la demande: 05.04.2017
(73) Titulaire: Novastep, 35760 St Gregoire (FR)
(72) Inventeur: GIROD, Loïc, 35580 Goven (FR); GAZONNET, Lilian, 01000 Bourg en Bresse (FR); LARIVIERE, Jean, 59290 Wasquehal (FR); RTAIMATE, Mohamed, 59273 Peronne en Melantois (FR)
(74) Mandataire: Chevalier, Renaud Philippe
(86) Numéro de dépôt international: PCT/FR2015/051411
(87) Numéro de publication internationale: WO 2015/185828

(56) Documents cités:
- EP-A1- 0 442 629
- EP-A1- 0 524 617
- EP-A1- 0 598 561
- WO-A1-2005/087440
- WO-A1-2010/094847
- DE-A1- 10 107 751
- FR-A1- 2 455 952

## Description

La présente invention concerne un organe de serrage, par exemple un embout de tournevis, pour serrer une vis à empreinte hexalobulaire. De plus, la présente invention concerne une vis à empreinte hexalobulaire, communément appelée vis hexalobulaire ou vis Torx ou vis-étoile.

La présente invention peut s'appliquer à tout domaine mettant en œuvre une vis hexalobulaire et un organe de serrage, par exemple un embout de tournevis, pour serrer une telle vis. En particulier, la présente invention peut aussi s'appliquer au domaine de la chirurgie de réparation osseuse, notamment pour serrer et fixer une vis d'ostéosynthèse dans un fragment d'os à stabiliser. Plus particulièrement, la présente invention peut s'appliquer à la chirurgie des extrémités (pieds, chevilles, mains, poignets).

FR2455952A1 décrit un embout de tournevis formant organe de serrage d'une vis hexalobulaire d'ostéosynthèse. Cet embout de tournevis comprend six lobes convexes et six lobes concaves répartis en alternance autour d'un axe central de sorte que chaque lobe concave raccorde deux lobes convexes respectifs. Grâce à ses six lobes convexes et à ses six lobes concaves, l'embout de tournevis de FR2455952A1 peut être positionné dans l'empreinte hexalobulaire de façon à serrer la vis hexalobulaire.

Afin de faciliter la manipulation et la préhension de la vis hexalobulaire, les six lobes convexes de l'embout de tournevis de FR2455952A1 sont coniques. La conicité des lobes convexes induit, à l'interface entre vis et tournevis, des frottements permettant la retenue de la vis hexalobulaire sur l'embout de tournevis. Le document EP 0 442 629 A1 divulgue aussi une organe de serrage de l'art antérieur.

Par exemple, lors d'un acte chirurgical, le chirurgien doit positionner la vis hexalobulaire d'ostéosynthèse sur le site d'implantation osseuse, puis connecter le tournevis et enfin serrer la vis hexalobulaire d'ostéosynthèse au moyen de l'embout de tournevis et avec un couple de serrage suffisant pour introduire dans l'os la vis hexalobulaire d'ostéosynthèse.

Cependant, comme l'ajustement de la conicité des lobes s'avère difficile, les frottements de retenue de la vis hexalobulaire peuvent varier d'un embout de tournevis à l'autre. Inversement, ces frottements de retenue peuvent varier d'une vis hexalobulaire à l'autre. En fonction de ces frottements de retenue variables, l'intensité de l'effort à fournir pour déconnecter la liaison entre vis et tournevis est parfois trop importante, parfois trop faible.

Lorsque la retenue est trop importante, l'opérateur doit appliquer un effort excessif à l'interface entre vis et tournevis, ce qui conduit à une usure prématurée de l'embout de tournevis ou à un endommagement de l'empreinte hexalobulaire de la vis qui rend difficile voire impossible une future extraction de cette vis. Inversement, lorsque la retenue est trop faible, il y a un risque de déconnexion intempestive de la liaison entre vis et tournevis, donc de chute de la vis hexalobulaire. Dans le cas d'une vis d'ostéosynthèse, la chute de la vis hexalobulaire hors du champ opératoire lui fait perdre son caractère stérile.

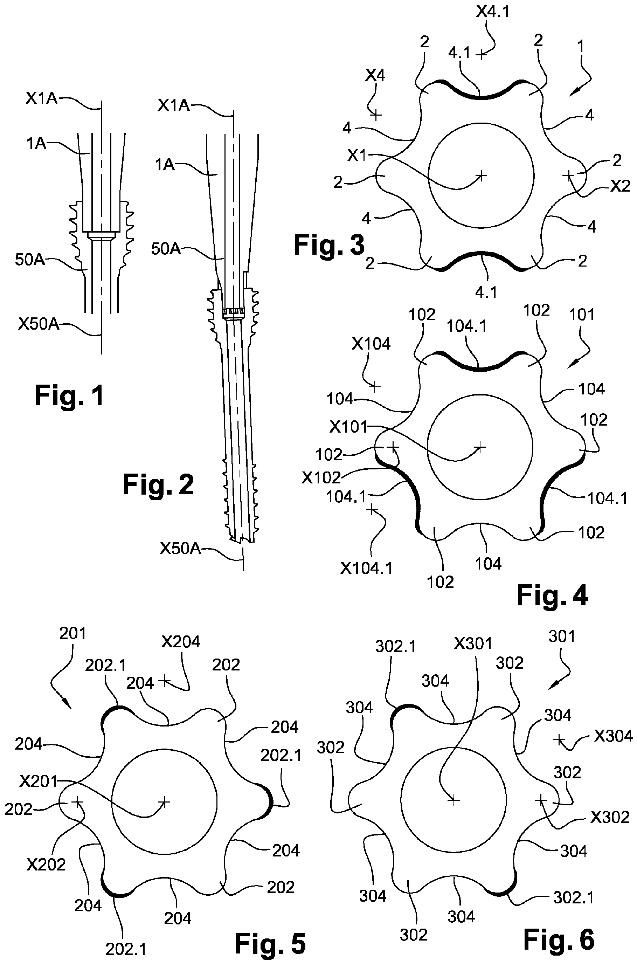

Par ailleurs, comme dans l'exemple de la figure 1, lorsque l'axe X50A de la vis hexalobulaire 50A et l'axe central X1A de l'embout de tournevis 1A sont alignés, la vis hexalobulaire 50A est entraînée en rotation suivant une trajectoire circulaire de diamètre minimal, ce qui minimise l'effort nécessaire à l'introduction de la vis hexalobulaire 50A dans l'os et le volume osseux nécessaire au logement de la vis hexalobulaire 50A.

Cependant, comme dans l'exemple de la figure 2, avec un embout de tournevis 1A de l'art antérieur, il est fréquent qu'une différence de conicité entre les lobes convexes provoque un défaut d'alignement de l'axe X50A de la vis hexalobulaire 50A et de l'axe central X1A de l'embout de tournevis 1A. En raison de ce défaut d'alignement, la vis hexalobulaire 50A est entraînée en rotation suivant une trajectoire qui n'est pas circulaire, ce qui augmente l'effort nécessaire à l'introduction de la vis hexalobulaire 50A, ainsi que le volume osseux nécessaire au logement de la vis hexalobulaire 50A.

En outre, comme un embout de tournevis est réutilisable, il est soumis à une usure qui est fonction du nombre d'utilisations. Lorsque la retenue de la vis dépend uniquement de la conicité des lobes convexes, plus l'embout de tournevis est utilisé, plus la surface des lobes convexes est aplanie par matage, ce qui dégrade la force de retenue des vis hexalobulaires sur cet embout de tournevis usagé.

La présente invention a notamment pour but de résoudre, en tout ou partie, les problèmes mentionnés ci-avant, en fournissant un organe de serrage et une vis à empreinte hexalobulaire permettant i) d'assurer de manière fiable et durable l'alignement entre l'axe des vis et l'axe de l'organe de serrage, ii) de produire continuellement une force de retenue fiable et répétitive, et iii) d'augmenter la durée de vie de l'organe de serrage ou de la vis hexalobulaire.

Dans ce but, l'invention a pour objet un organe de serrage, pour serrer une vis à empreinte hexalobulaire, par exemple une vis d'ostéosynthèse, l'organe de serrage comprenant six lobes convexes et six lobes concaves répartis en alternance autour d'un axe central de sorte que chaque lobe concave raccorde deux lobes convexes consécutifs autour de l'axe central, l'organe de serrage étant caractérisé en ce que :
i) les lobes convexes comprennent :
   - au moins trois lobes convexes cylindriques, chaque lobe convexe cylindrique étant défini sensiblement par une portion de cylindre respective ayant un axe substantiellement parallèle à l'axe central, et
   - au moins deux lobes convexes coniques, chaque lobe convexe conique étant défini sensiblement par une portion de cône respective ayant un axe substantiellement parallèle à l'axe central, deux lobes convexes coniques respectifs étant séparés par au moins un lobe convexe cylindrique,
et/ou en ce que :
ii) les lobes concaves comprennent :
- au moins trois lobes concaves cylindriques, chaque lobe concave cylindrique étant défini sensiblement par une portion de cylindre respective ayant un axe substantiellement parallèle à l'axe central, et
- au moins deux lobes concaves coniques, chaque lobe concave conique étant défini sensiblement par une portion de cône respective ayant un axe substantiellement parallèle à l'axe central, deux lobes concaves coniques respectifs étant séparés par au moins un lobe concave cylindrique.

Dans la présente demande, le terme « cylindre » désigne une surface tridimensionnelle engendrée par une droite génératrice qui se déplace parallèlement à un axe en s'appuyant sur une base. Un cylindre peut avoir une base en forme de cercle ou d'une autre courbe, voire de polygone.

Dans la présente demande, le terme « cône » désigne une surface tridimensionnelle engendrée par une droite génératrice mobile, passant par un point fixe (sommet) en s'appuyant sur une base. Un cône peut avoir une base en forme de cercle ou d'une autre courbe, voire de polygone.

En particulier, l'organe de serrage peut former un embout de tournevis.

Dans la présente demande, le terme « lobe » désigne notamment une portion de l'organe de serrage qui est située à la surface de l'organe de serrage, qui a généralement une forme arrondie et qui est délimitée nettement par des éléments voisins. En général, la forme arrondie d'un lobe résulte d'une géométrie de révolution. Chaque lobe convexe a une forme convexe, alors que chaque lobe concave a une forme concave.

Ainsi, un tel organe de serrage permet i) d'assurer de manière fiable et durable l'alignement entre l'axe des vis et l'axe de l'organe de serrage, ii) tout en produisant continuellement une force de retenue fiable et répétitive, et iii) en augmentant la durée de vie de l'organe de serrage ou de la vis hexalobulaire.

En particulier, l'interposition d'au moins un lobe convexe et/ou concave cylindrique entre deux des lobes convexes et/ou concaves coniques consécutifs permet un certain éloignement des lobes convexes ou concaves coniques, ce qui garantit l'alignement de l'axe central avec la direction axiale.

Selon un mode de réalisation de l'invention, les lobes convexes comprennent au moins trois lobes convexes cylindriques et au moins deux lobes convexes coniques, et les lobes concaves sont constitués de six lobes concaves cylindriques.

Ainsi, l'organe de serrage est relativement simple à fabriquer, car seuls des lobes convexes sont coniques, tandis que tous les lobes concaves sont cylindriques.

Selon un mode de réalisation de l'invention, les lobes concaves comprennent au moins trois lobes concaves cylindriques et au moins deux lobes concaves coniques, et les lobes convexes sont constitués de six lobes convexes cylindriques.

Ainsi, l'organe de serrage est relativement simple à fabriquer, car seuls des lobes concaves sont coniques, tandis que tous les lobes convexes sont cylindriques.

Selon un mode de réalisation de l'invention, les lobes concaves comprennent seulement deux lobes concaves coniques, et/ou les lobes convexes comprennent seulement deux lobes convexes coniques.

Ainsi, l'organe de serrage est relativement simple à fabriquer, car seuls deux lobes convexes et/ou seuls deux lobes concaves sont coniques.

Selon un mode de réalisation de l'invention, les lobes concaves comprennent trois lobes concaves coniques, et/ou les lobes convexes comprennent trois lobes convexes coniques.

En d'autres termes, l'organe de serrage comprend i) trois lobes concaves coniques et/ou trois lobes convexes coniques, placés respectivement aux trois sommets d'un triangle équilatéral, ainsi que ii) trois lobes concaves cylindriques et/ou trois lobes convexes cylindriques.

Ainsi, ces trois lobes concaves coniques et/ou trois lobes convexes coniques permettent de réaliser un alignement très précis de l'axe central avec la direction axiale. En effet, ces trois lobes concaves coniques et/ou trois lobes convexes coniques améliorent le positionnement tridimensionnel de l'organe de serrage par rapport à la vis à empreinte hexalobulaire.

Selon un mode de réalisation de l'invention, chaque lobe convexe conique et/ou chaque lobe concave conique présente un demi-angle au sommet compris entre 1 degrés et 5 degrés.

Ainsi, un tel demi-angle au sommet, relativement petit, permet d'accomplir la fonction de retenue de la vis hexalobulaire sans nécessiter un effort excessif de la part de l'opérateur pour déconnecter l'organe de serrage d'une vis à empreinte hexalobulaire.

Selon un mode de réalisation de l'invention, chaque lobe concave conique présente, en section dans un plan contenant l'axe central, un profil comprenant un segment rectiligne qui s'étend depuis la face libre de l'organe de serrage et qui est incliné sur l'axe central, et/ou chaque lobe convexe conique présente, en section dans un plan contenant l'axe central, un profil comprenant un segment rectiligne qui s'étend depuis la face libre de l'organe de serrage et qui est incliné sur l'axe central.

Dans le cas où le profil de chaque lobe concave conique est entièrement rectiligne, chaque lobe concave a une forme parfaitement conique. Ainsi, un tel profil à segment rectiligne est relativement simple à fabriquer, par exemple au moyen d'un centre d'usinage numérique à quatre axes.

Selon un mode de réalisation de l'invention, chaque segment rectiligne a une longueur comprise entre 0,3 mm et 4,0 mm.

Ainsi, de tels segments rectilignes permettent le centrage de l'embout de tournevis dans l'empreinte hexalobulaire de la vis.

Selon un mode de réalisation de l'invention, chaque intersection entre chaque segment rectiligne et la face libre est située à une distance représentant entre 30% et 80% de la distance séparant l'axe central et le sommet d'un lobe convexe, les distances étant mesurées perpendiculairement à l'axe central.

Ainsi, une telle distance permet d'obtenir une force de retenue relativement grande.

Selon un mode de réalisation de l'invention, ledit profil de chaque lobe concave conique comprend en outre un segment curviligne, par exemple circulaire, s'étendant tangentiellement à un segment rectiligne respectif dans un plan contenant l'axe central, le segment curviligne ayant de préférence un rayon de courbure compris entre 2 mm et 8 mm et une longueur comprise entre 0,5 mm et 2,0 mm, et/ou ledit profil de chaque lobe convexe conique comprend en outre un segment curviligne s'étendant tangentiellement à un segment rectiligne respectif dans un plan contenant l'axe central.

En d'autres termes, le profil de chaque lobe concave conique est composé, à partir de la face libre de l'organe de serrage, d'un segment rectiligne et d'un segment curviligne. De tels profils sont donc sensiblement coniques, mais pas parfaitement coniques.

Ainsi, de tels profils coniques garantissent une retenue efficace et durable malgré l'usure de l'organe de serrage après de nombreuses utilisation. En effet, même en cas de matage des lobes coniques, la retenue sera obtenue par plusieurs contacts ponctuels entre, d'une part, les lobes externes cylindriques de la vis à empreinte hexalobulaire et, d'autre part, le segment curviligne, des lobes concaves de l'organe de serrage.

Selon une variante de l'invention, l'organe de serrage est composé d'un acier inoxydable, par exemple de nuance X15TN (X40CrMoVN16-2 suivant la codification W.Nr. 1.4123).

Par ailleurs, la présente invention a pour objet une vis à empreinte hexalobulaire, par exemple vis d'ostéosynthèse, la vis à empreinte hexalobulaire comprenant six lobes externes et six lobes internes répartis en alternance autour d'une direction axiale de sorte que chaque lobe interne raccorde deux lobes externes respectifs,
la vis à empreinte hexalobulaire étant caractérisée en ce que :
i) les lobes externes comprennent :
   - au moins trois lobes externes cylindriques, chaque lobe externe cylindrique étant défini sensiblement par un cylindre respectif ayant un axe substantiellement parallèle à la direction axiale, et
   - au moins deux lobes externes coniques, chaque lobe externe conique étant défini sensiblement par un cône respectif ayant un axe substantiellement parallèle à la direction axiale, deux lobes externes coniques respectifs étant séparés par au moins un lobe externe cylindrique,
et en ce que :
ii) les lobes internes comprennent :
- au moins trois lobes internes cylindriques, chaque lobe interne cylindrique étant défini sensiblement par un cylindre respectif ayant un axe substantiellement parallèle à la direction axiale, et
- au moins deux lobes internes coniques, chaque lobe interne conique étant défini sensiblement par un cône respectif ayant un axe substantiellement parallèle à la direction axiale, deux lobes internes coniques respectifs étant séparés par au moins un lobe interne cylindrique.

Ainsi, une telle vis à empreinte hexalobulaire permet i) d'assurer de manière fiable et durable l'alignement entre l'axe des vis et l'axe de l'organe de serrage, ii) tout en produisant continuellement une force de retenue fiable et répétitive, et iii) en augmentant la durée de vie de l'organe de serrage ou de la vis à empreinte hexalobulaire.

En particulier, l'interposition d'au moins un lobe convexe et/ou concave cylindrique entre deux des lobes convexes et/ou concaves coniques consécutifs permet un certain éloignement des lobes convexes ou concaves coniques, ce qui garantit l'alignement de la direction axiale avec l'axe central de l'organe de serrage.

Selon un mode de réalisation de l'invention, les lobes externes comprennent au moins trois lobes externes cylindriques et au moins deux lobes externes coniques, et les lobes internes sont constitués de six lobes internes cylindriques.

Ainsi, la vis à empreinte hexalobulaire est relativement simple à fabriquer, car seuls des lobes externes sont coniques, tandis que tous les lobes internes sont cylindriques.

Selon un mode de réalisation de l'invention, les lobes internes comprennent au moins trois lobes internes cylindriques et au moins deux lobes internes coniques, et les lobes externes sont constitués de six lobes externes cylindriques.

Ainsi, la vis à empreinte hexalobulaire est relativement simple à fabriquer, car seuls des lobes internes sont coniques, tandis que tous les lobes externes sont cylindriques.

Selon une variante de l'invention, la vis à empreinte hexalobulaire est composée d'un alliage de titane, par exemple un alliage de titane TA6V.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une coupe d'un organe de serrage de l'art antérieur en cours de serrage d'une vis à empreinte hexalobulaire, dans une configuration d'alignement ; la figure 1 a été décrite ci-avant en relation avec l'art antérieur ;
- la figure 2 est une coupe d'un organe de serrage de l'art antérieur en cours de serrage d'une vis à empreinte hexalobulaire, dans une configuration de défaut d'alignement ; la figure 2 a été décrite ci-avant en relation avec l'art antérieur ;
- la figure 3 est une vue de face d'une partie d'un organe de serrage conforme à un premier mode de réalisation de l'invention ;
- la figure 4 est une vue de face d'une partie d'un organe de serrage conforme à un deuxième mode de réalisation de l'invention ;
- la figure 5 est une vue de face d'une partie d'un organe de serrage conforme à un troisième mode de réalisation de l'invention ;
- la figure 6 est une vue de face d'une partie d'un organe de serrage conforme à un quatrième mode de réalisation de l'invention ;
- la figure 7 est une vue de côté de l'organe de serrage de la figure 3 au cours d'une étape d'un procédé de fabrication ;
- la figure 8 est une vue en perspective de l'organe de serrage de la figure 7 au cours d'une étape d'un procédé de fabrication ;
- la figure 9 est une vue de côté de l'organe de serrage de la figure 7 au cours d'une étape d'un procédé de fabrication ;
- la figure 10 est une vue en perspective de l'organe de serrage des figures 7, 8 et 9 issu du procédé de fabrication ;
- la figure 11 est une coupe d'une partie de l'organe de serrage neuf en cours de serrage d'une vis à empreinte hexalobulaire ;
- la figure 12 est une coupe d'une partie de l'organe de serrage usé en cours de serrage d'une vis à empreinte hexalobulaire ;
- la figure 13 est une vue de face d'une partie d'une vis à empreinte hexalobulaire conforme à un cinquième mode de réalisation de l'invention ; et
- la figure 14 est une vue de face d'une partie d'une vis à empreinte hexalobulaire conforme à un sixième mode de réalisation de l'invention.

La figure 3 illustre un organe de serrage 1 conforme à un premier mode de réalisation de l'invention, pour serrer une vis à empreinte hexalobulaire, en l'occurrence une vis d'ostéosynthèse. Dans l'exemple de la figure 3, l'organe de serrage 1 forme un embout de tournevis. L'organe de serrage 1 est ici composé d'un acier inoxydable, par exemple de nuance inoxydable X15TN.

L'organe de serrage 1 comprend six lobes convexes 2 et six lobes concaves 4 et 4.1 répartis en alternance autour d'un axe central X1 de sorte que chaque lobe concave 4 ou 4.1 raccorde deux lobes convexes 2 consécutifs autour de l'axe central X1. En d'autres termes, deux lobes convexes 2 consécutifs quelconques sont raccordés par un lobe concave respectif 4 ou 4.1. Comme le montre la figure 3, chaque lobe convexe 2 est séparé du lobe concave voisin 4 ou 4.1 par un point d'inflexion respectif.

Dans l'exemple de la figure 3, les lobes concaves comprennent quatre lobes concaves cylindriques 4 et seulement deux lobes concaves coniques 4.1. Les lobes convexes comprennent ici six lobes convexes cylindriques 2.

Chaque lobe concave cylindrique 4 est défini sensiblement par une portion de cylindre respective ayant un axe X4 qui est substantiellement parallèle à l'axe central X1.

Chaque lobe concave conique 4.1 est défini sensiblement par une portion de cône respective, qui a un axe X4.1 substantiellement parallèle à l'axe central X1 et qui présente un demi-angle A au sommet environ égal à 1,75 degrés, soit un angle de cône de 3,5 degrés. De manière connue en soi, le demi-angle au sommet A caractérise la conicité d'un cône, c'est-à-dire l'inclinaison de ses génératrices sur son axe. Les deux lobes concaves coniques 4.1 sont séparés par deux lobes concaves cylindriques 4.

La figure 4 illustre un organe de serrage 101 conforme à un deuxième mode de réalisation de l'invention, pour serrer une vis à empreinte hexalobulaire, en l'occurrence une vis d'ostéosynthèse. Dans l'exemple de la figure 4, l'organe de serrage 101 forme un embout de tournevis. L'organe de serrage 101 est ici composé d'un acier inoxydable.

L'organe de serrage 101 comprend six lobes convexes 102 et six lobes concaves 104 et 104.1 répartis en alternance autour d'un axe central X101 de sorte que chaque lobe concave 104 ou 104.1 raccorde deux lobes convexes 102 consécutifs autour de l'axe central X101. En d'autres termes, deux lobes convexes 102 consécutifs quelconques sont raccordés par un lobe concave respectif 104 ou 104.1. Comme le montre la figure 4, chaque lobe convexe 102 est séparé du lobe concave voisin 104 ou 104.1 par un point d'inflexion respectif.

À la différence de l'organe de serrage 1, dans l'exemple de la figure 4, les lobes concaves comprennent trois lobes concaves cylindriques 104, trois lobes concaves coniques 104.1. Les lobes convexes comprennent ici six lobes convexes cylindriques 102.

Chaque lobe concave cylindrique 104 est défini sensiblement par une portion de cylindre respective ayant un axe X104 qui est substantiellement parallèle à l'axe central X101. Chaque portion de cylindre a ici une base en forme d'arc d'ellipse.

Chaque lobe concave conique 104.1 est défini sensiblement par une portion de cône respective, qui a un axe X104.1 substantiellement parallèle à l'axe central X101 et qui présente un demi-angle au sommet environ égal à 1,75 degrés. Chaque portion de cône a ici une base en forme d'arc d'ellipse. Deux lobes convexes coniques respectifs 104.1 sont séparés par un lobe concave cylindrique 104.

Les six lobes convexes 102 sont répartis uniformément autour de l'axe central X101. Les six lobes concaves 104 sont répartis uniformément autour de l'axe central X101.

Comme les lobes concaves et les lobes convexes sont répartis uniformément autour de l'axe central X101, les axes X104.1 des trois lobes concaves coniques 104.1 se trouvent placés respectivement aux trois sommets d'un triangle équilatéral centré sur l'axe central X101. En d'autres termes, les portions centrales de deux lobes concaves coniques 104.1 consécutifs sont séparés par un angle de 120 degrés.

La figure 5 illustre un organe de serrage 201 conforme à un troisième mode de réalisation de l'invention, pour serrer une vis à empreinte hexalobulaire, en l'occurrence une vis d'ostéosynthèse. Dans l'exemple de la figure 5, l'organe de serrage 201 forme un embout de tournevis. L'organe de serrage 201 est ici composé d'un acier inoxydable.

L'organe de serrage 201 comprend six lobes convexes 202 et 202.1 et six lobes concaves 204 répartis en alternance autour d'un axe central X201 de sorte que chaque lobe concave 204 raccorde deux lobes convexes 202 ou 202.1 consécutifs autour de l'axe central X201. En d'autres termes, deux lobes convexes 202 ou 202.1 consécutifs quelconques sont raccordés par un lobe concave respectif 204. Comme le montre la figure 5, chaque lobe convexe 202 ou 202.1 est séparé du lobe concave voisin 204 par un point d'inflexion respectif.

À la différence de l'organe de serrage 101, dans l'exemple de la figure 5, les lobes convexes comprennent trois lobes convexes cylindriques 202 et trois lobes convexes coniques 202.1. Les lobes concaves comprennent ici six lobes concaves cylindriques 202.

Chaque lobe convexe cylindrique 202 est défini sensiblement par une portion de cylindre respective ayant un axe X202 qui est substantiellement parallèle à l'axe central X201. Chaque portion de cylindre a ici une base en forme d'arc d'ellipse.

Chaque lobe concave conique 202.1 est défini sensiblement par une portion de cône respective, qui a un axe X202.1 substantiellement parallèle à l'axe central X201 et qui présente un demi-angle au sommet environ égal à 1,75 degrés. Chaque portion de cône a ici une base en forme d'arc d'ellipse. Deux lobes convexes coniques respectifs 202.1 sont séparés par un lobe convexe cylindrique 202.

Les six lobes convexes 202 sont répartis uniformément autour de l'axe central X201. Les six lobes concaves 204 sont répartis uniformément autour de l'axe central X201.

Comme les lobes concaves et les lobes convexes sont uniformément répartis autour de l'axe central X201, les axes X204.1 des trois lobes convexes coniques 202.1 se trouvent placés respectivement aux trois sommets d'un triangle équilatéral centré sur l'axe central X101. En d'autres termes, les portions centrales de deux lobes convexes coniques 202.1 consécutifs sont séparés par un angle de 120 degrés.

La figure 6 illustre un organe de serrage 301 conforme à un quatrième mode de réalisation de l'invention, pour serrer une vis à empreinte hexalobulaire, en l'occurrence une vis d'ostéosynthèse. Dans l'exemple de la figure 6, l'organe de serrage 301 forme un embout de tournevis. L'organe de serrage 301 est ici composé d'un acier inoxydable.

L'organe de serrage 301 comprend six lobes convexes 302 et 302.1 et six lobes concaves 304 répartis en alternance autour d'un axe central X301 de sorte que chaque lobe concave 304 raccorde deux lobes convexes 302 ou 302.1 consécutifs autour de l'axe central X301. En d'autres termes, deux lobes convexes 302 ou 302.1 consécutifs quelconques sont raccordés par un lobe concave respectif 304. Comme le montre la figure 6, chaque lobe convexe 302 ou 302.1 est séparé du lobe concave voisin 304 par un point d'inflexion respectif.

À la différence de l'organe de serrage 201, dans l'exemple de la figure 6, les lobes convexes comprennent quatre lobes convexes cylindriques 302 et seulement deux lobes convexes coniques 302.1. Les lobes concaves comprennent ici six lobes concaves cylindriques 102.

Chaque lobe convexe cylindrique 302 est défini sensiblement par une portion de cylindre respective ayant un axe X302 qui est substantiellement parallèle à l'axe central X301. Chaque portion de cylindre a ici une base en forme d'arc d'ellipse.

Chaque lobe concave conique 302.1 est défini sensiblement par une portion de cône respective, qui a un axe X302.1 substantiellement parallèle à l'axe central X301 et qui présente un demi-angle au sommet environ égal à 1,75 degrés. Chaque portion de cône a ici une base en forme d'arc d'ellipse. Les deux lobes convexes coniques respectifs 302.1 sont séparés par deux lobes convexes cylindriques 302.

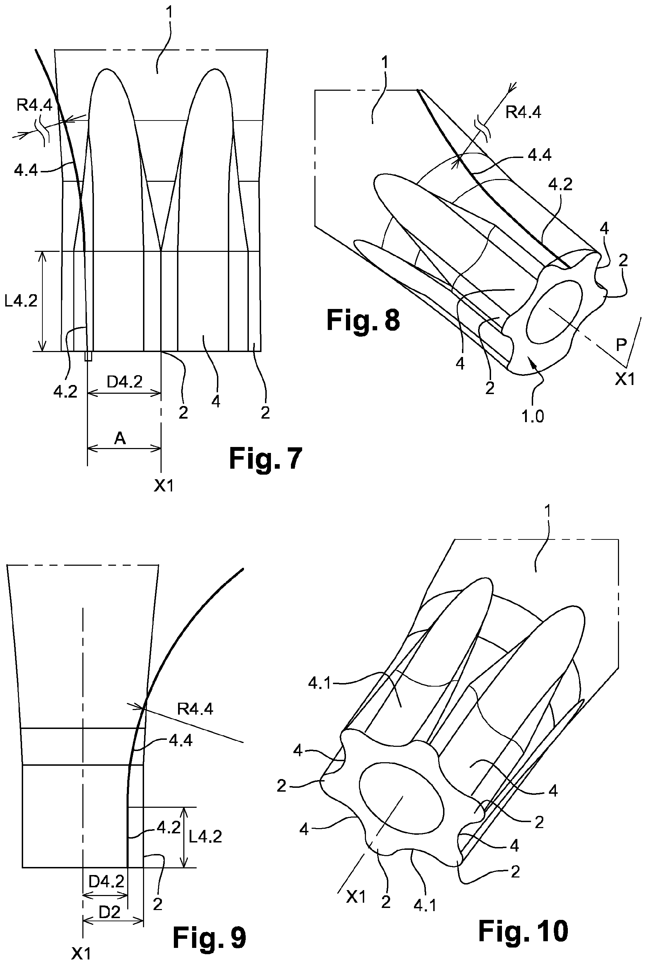

Les figures 7, 8, 9 et 10 illustrent des étapes d'un procédé de fabrication de l'organe de serrage 1. Le procédé de fabrication peut par exemple mettre en œuvre un centre numérique d'usinage à quatre axes.

Comme le montrent les figures 7 à 10, chaque lobe concave conique 4.1 présente, en section dans un plan P (par exemple le plan de la figure 7 ou 9) contenant l'axe central X1, un profil comprenant :
- un segment rectiligne 4.2, et
- un segment curviligne 4.4.

En d'autres termes, le profil de chaque lobe concave conique 4.1 est composé, à partir de la face libre 1.0 de l'organe de serrage 1, du segment rectiligne 4.2 et du segment curviligne 4.4.

Le segment rectiligne 4.2 s'étend depuis la face libre 1.0 de l'organe de serrage 1. Le segment rectiligne 4.2 est incliné sur l'axe central X1 du demi-angle au sommet A, qui est matérialisé à la figure 7 et qui est environ égal à 1,75 degrés. Le segment rectiligne a une longueur L4.4 environ égale à 1,0 mm. Le segment rectiligne 4.2 définit une portion parfaitement conique pour un lobe concave conique 4.1.

Comme le montre la figure 9, l'intersection entre le segment rectiligne 4.2 et la face libre 1.0 est située à une distance D4.2 représentant environ 70% de la distance D2 séparant l'axe central X1 et le sommet d'un lobe convexe 2, les distances étant mesurées perpendiculairement à l'axe central X1.

Le segment curviligne 4.4 est ici circulaire. Le segment curviligne 4.4 s'étend tangentiellement au segment rectiligne 4.2 dans le plan Procédé contenant l'axe central X1. Le segment curviligne 4.4 a ici un rayon de courbure R4.4 environ égal à 5 mm et une longueur environ égale à 2 mm.

La figure 8 illustre l'organe de serrage au cours d'une étape du procédé de fabrication. Comme le montre la figure 8, cette étape précède l'usinage des deux lobes concaves coniques 4.1, car la matière n'a pas encore été enlevée au niveau des deux lobes concaves coniques 4.1. Avantageusement, l'usinage est réalisé simultanément pour les deux lobes concaves coniques 4.1. Cela permet d'usiner les deux lobes concaves coniques 4.1 de manière très symétrique.

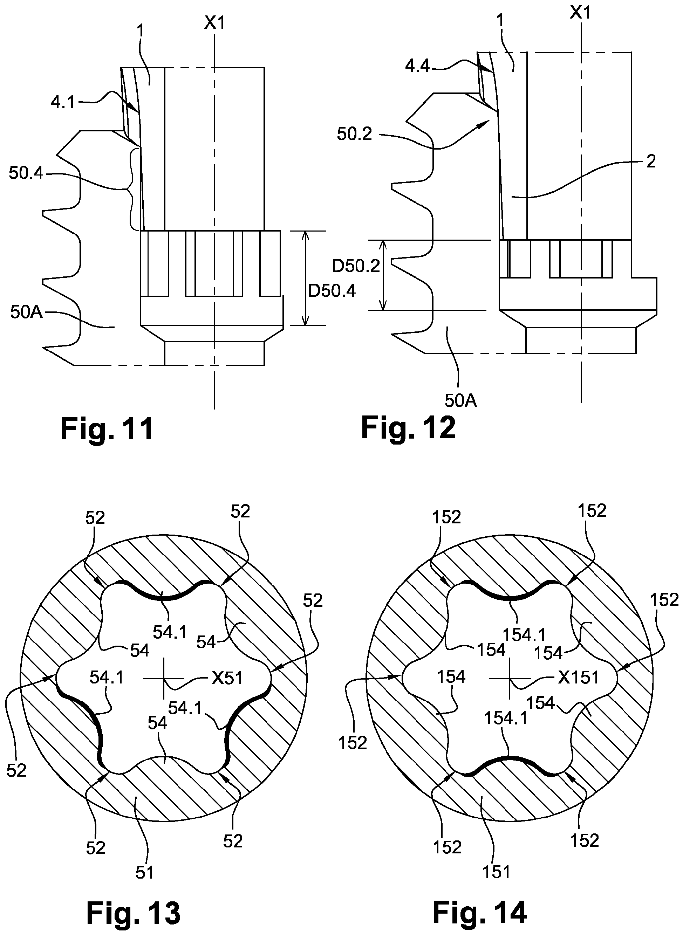

En service, comme le montrent les figures 11 et 12, un opérateur peut serrer des vis à empreintes hexalobulaires au moyen des organes de serrage 1, 101, 201 ou 301.

L'organe de serrage 1, 101, 201 ou 301 permet i) d'assurer de manière fiable et durable l'alignement entre les axes des vis et l'axe central X1 de l'organe de serrage 1, ii) tout en produisant continuellement (neuf puis usé) une force de retenue fiable et répétitive, et iii) en augmentant la durée de vie de l'organe de serrage 1 ou d'une vis à empreinte hexalobulaire 50A, y compris quand l'organe de serrage 1 est usé. En particulier, l'interposition des lobes concaves cylindriques 4 entre les deux lobes concaves coniques 4.1 garantit l'alignement de l'axe central X1 avec la direction axiale de la vis à empreinte hexalobulaire 50A.

La figure 11 est une coupe d'une partie de l'organe de serrage 1 à l'état neuf au cours d'un serrage de la vis à empreinte hexalobulaire 50A.La figure 12 est une coupe d'une partie de l'organe de serrage 1 à l'état usé au cours d'un serrage de la vis à empreinte hexalobulaire 50A.

Lorsque l'organe de serrage 1 est à l'état neuf (figure 11), les lobes concaves coniques 4.1 ont leur forme conique intègre. La force de retenue et la cohésion entre l'organe de serrage 1 et la vis à empreinte hexalobulaire 50A sont produites par les contacts d'interférence qui interviennent au niveau d'une région 50.4 entre les surfaces coniques des lobes concaves coniques 4.1 et les surfaces cylindriques des lobes externes cylindriques correspondants de la vis à empreinte hexalobulaire 50A. Dans cette configuration, une première distance D50.4 sépare la face libre 1.0 du fond de l'empreinte hexalobulaire de la vis 50A.

Lorsque l'organe de serrage 1 est à l'état usé (figure 12), les lobes concaves coniques 4.1 sont matés, partiellement ou totalement. La force de retenue et la cohésion entre l'organe de serrage 1 et la vis à empreinte hexalobulaire 50A sont produites par les contacts d'interférence qui interviennent sur des contacts ponctuels entre les lobes externes cylindriques de la vis à empreinte hexalobulaire 50A et le segment curviligne 4.4 du profil des lobes concaves coniques 4.1. Dans cette configuration, une deuxième distance D50.2 sépare la face libre 1.0 du fond de l'empreinte hexalobulaire de la vis 50A. Comme le montre une comparaison des figures 11 et 12, la deuxième distance D50.2 est inférieure à la première distance D50.4.

Ainsi, les profils des lobes concaves coniques 4.1, grâce à leurs segments curvilignes 4.4, garantissent une retenue efficace et durable malgré l'usure de l'organe de serrage 1 après de nombreuses utilisation.

La figure 13 illustre une vis à empreinte hexalobulaire 51 conforme à un cinquième mode de réalisation de l'invention. Dans l'exemple de la figure 13, la vis à empreinte hexalobulaire 51 forme une vis d'ostéosynthèse. La vis à empreinte hexalobulaire 51 est ici composée d'un alliage de titane TA6V. La vis à empreinte hexalobulaire 51 peut être serrée par exemple au moyen de l'organe de serrage 1.

La vis à empreinte hexalobulaire 51 comprenant six lobes externes 52 et six lobes internes 54 et 54.1 répartis en alternance autour d'une direction axiale X51 de sorte que chaque lobe interne 54 ou 54.1 raccorde deux lobes externes respectifs 52. En d'autres termes, deux lobes externes 52 consécutifs quelconques sont raccordés par un lobe interne respectif 54 ou 54.1. Comme le montre la figure 13, chaque lobe externe 52 est séparé du lobe interne voisin 54 ou 54.1 par un point d'inflexion respectif.

Dans l'exemple de la figure 13, les lobes internes 54 comprennent trois lobes internes cylindriques 54 et trois lobes internes coniques 54.1. Les lobes externes comprennent ici six lobes externes cylindriques 52.

Chaque lobe interne cylindrique 52 est défini sensiblement par un cylindre respectif ayant un axe substantiellement parallèle à la direction axiale X51.

Chaque lobe interne conique 54.1 est défini sensiblement par un cône respectif, qui a un axe substantiellement parallèle à la direction axiale X51 et qui présente un demi-angle au sommet environ égal à 3 degrés. Les trois lobes internes coniques 54.1 sont séparés deux à deux par au moins un lobe interne cylindrique 52.

La vis à empreinte hexalobulaire 51 permet i) d'assurer de manière fiable et durable l'alignement entre la direction axiale X51 et l'axe d'un organe de serrage, ii) tout en produisant continuellement une force de retenue fiable et répétitive, et iii) en augmentant la durée de vie de l'organe de serrage ou de la vis à empreinte hexalobulaire 51.

La figure 14 illustre une vis à empreinte hexalobulaire 151 conforme à un sixième mode de réalisation de l'invention. Dans l'exemple de la figure 14, la vis à empreinte hexalobulaire 151 forme une vis d'ostéosynthèse. La vis à empreinte hexalobulaire 151 est ici composée d'un alliage de titane TA6V. La vis à empreinte hexalobulaire 151 peut être serrée par exemple au moyen de l'organe de serrage 1.

La vis à empreinte hexalobulaire 151 comprenant six lobes externes 152 et six lobes internes 154 et 154.1 répartis en alternance autour d'une direction axiale X151 de sorte que chaque lobe interne 154 ou 154.1 raccorde deux lobes externes respectifs 152. En d'autres termes, deux lobes externes 152 consécutifs quelconques sont raccordés par un lobe interne respectif 154 ou 154.1. Comme le montre la figure 14, chaque lobe externe 152 est séparé du lobe interne voisin 54 ou 154.1 par un point d'inflexion respectif.

Dans l'exemple de la figure 14, les lobes internes 154 comprennent quatre lobes internes cylindriques 154 et seulement deux lobes internes coniques 154.1. Les lobes externes comprennent ici six lobes externes cylindriques 152.

Chaque lobe interne cylindrique 152 est défini sensiblement par un cylindre respectif ayant un axe substantiellement parallèle à la direction axiale X151.

Chaque lobe interne conique 154.1 est défini sensiblement par un cône respectif, qui a un axe substantiellement parallèle à la direction axiale X151 et qui présente un demi-angle au sommet environ égal à 1,75 degrés. Les deux lobes internes coniques 154.1 sont séparés deux à deux par deux lobes internes cylindriques 152.

La vis à empreinte hexalobulaire 151 permet i) d'assurer de manière fiable et durable l'alignement entre la direction axiale X151 et l'axe d'un organe de serrage, ii) tout en produisant continuellement une force de retenue fiable et répétitive, et iii) en augmentant la durée de vie de l'organe de serrage ou de la vis à empreinte hexalobulaire 151.

Bien entendu, la présente invention n'est pas limitée aux modes de réalisation particuliers décrits dans le présent

## Revendications

1. Organe de serrage (1 ; 101 ; 201 ; 301), pour serrer une vis à empreinte hexalobulaire, par exemple une vis d'ostéosynthèse, l'organe de serrage (1-301) comprenant six lobes convexes et six lobes concaves répartis en alternance autour d'un axe central (X1-X301) de sorte que chaque lobe concave raccorde deux lobes convexes consécutifs autour de l'axe central (X1-X301),
l'organe de serrage (1 ; 101 ; 201 ; 301) étant **caractérisé en ce que** :
i) les lobes convexes comprennent :
- au moins trois lobes convexes cylindriques (2 ; 102 ; 202 ; 302), chaque lobe convexe cylindrique (2-302) étant défini sensiblement par une portion de cylindre respective ayant un axe (X2-X302) substantiellement parallèle à l'axe central (X1-X301), et
- au moins deux lobes convexes coniques (202.1 ; 302.1), chaque lobe convexe conique (202.1 ; 302.1) étant défini sensiblement par une portion de cône respective ayant un axe substantiellement parallèle à l'axe central (X1-X301), deux lobes convexes coniques respectifs (202.1 ; 302.1) étant séparés par au moins un lobe convexe cylindrique (202 ; 302),
et/ou **en ce que** :
ii) les lobes concaves comprennent :
- au moins trois lobes concaves cylindriques (4 ; 104 ; 204 ; 304), chaque lobe concave cylindrique (4-304) étant défini sensiblement par une portion de cylindre respective ayant un axe (X4-X304) substantiellement parallèle à l'axe central (X1-X301), et
- au moins deux lobes concaves coniques (4.1 ; 104.1), chaque lobe concave conique (4.1 ; 104.1) étant défini sensiblement par une portion de cône respective ayant un axe (X4.1 ; X104.1) substantiellement parallèle à l'axe central (X1-X301), deux lobes concaves coniques respectifs (4.1 ; 104.1) étant séparés par au moins un lobe concave cylindrique (4-104).

2. Organe de serrage (201 ; 301) selon la revendication 1, dans lequel les lobes convexes comprennent au moins trois lobes convexes cylindriques (202 ; 302) et au moins deux lobes convexes coniques (202.1 ; 302.1), et dans lequel les lobes concaves sont constitués de six lobes concaves cylindriques (204 ; 304).

3. Organe de serrage (1 ; 101) selon la revendication 1, dans lequel les lobes concaves comprennent au moins trois lobes concaves cylindriques (4 ; 104) et au moins deux lobes concaves coniques (4.1 ; 104.1), et dans lequel les lobes convexes sont constitués de six lobes convexes cylindriques (2 ; 102).

4. Organe de serrage (1 / 301) selon l'une quelconque des revendications précédentes, dans lequel les lobes concaves comprennent seulement deux lobes concaves coniques (4.1), et/ou dans lequel les lobes convexes comprennent seulement deux lobes convexes coniques (302.1).

5. Organe de serrage (101 / 201) selon l'une quelconque des revendications précédentes, dans lequel les lobes concaves comprennent trois lobes concaves coniques (104.1), et/ou dans lequel les lobes convexes comprennent trois lobes convexes coniques (202.1).

6. Organe de serrage (1-301) selon l'une quelconque des revendications précédentes, dans lequel chaque lobe convexe conique (202.1 ; 302.1) et/ou chaque lobe concave conique (4.1 ; 104.1) présente un demi-angle (A) au sommet compris entre 1 degrés et 5 degrés.

7. Organe de serrage (1) selon l'une quelconque des revendications précédentes, dans lequel chaque lobe concave conique (4.1) présente, en section dans un plan (P) contenant l'axe central (X1), un profil comprenant un segment rectiligne (4.2) qui s'étend depuis la face libre (1.0) de l'organe de serrage (1) et qui est incliné sur l'axe central (X1), et/ou dans lequel chaque lobe convexe conique présente, en section dans un plan contenant l'axe central, un profil comprenant un segment rectiligne qui s'étend depuis la face libre de l'organe de serrage et qui est incliné sur l'axe central.

8. Organe de serrage (1) selon la revendication 7, dans lequel chaque segment rectiligne (4.2) a une longueur (L4.2) comprise entre 0,3 mm et 4,0 mm.

9. Organe de serrage (1) selon l'une quelconque des revendications 7 à 8, chaque intersection entre chaque segment rectiligne (4.2) et la face libre (1.0) est située à une distance (D4.2) représentant entre 30% et 80% de la distance (D2) séparant l'axe central (X1) et le sommet d'un lobe convexe (2), les distances étant mesurées perpendiculairement à l'axe central (X1).

10. Organe de serrage (1) selon l'une quelconque des revendications 7 à 9, dans lequel ledit profil de chaque lobe concave conique (4.1) comprend en outre un segment curviligne (4.4), par exemple circulaire, s'étendant tangentiellement à un segment rectiligne respectif (4.2) dans un plan (P) contenant l'axe central (X1), le segment curviligne (4.4) ayant de préférence un rayon de courbure (R4.4) compris entre 2 mm et 8 mm et une longueur comprise entre 0,5 mm et 2,0 mm, et/ou dans lequel ledit profil de chaque lobe convexe conique comprend en outre un segment curviligne s'étendant tangentiellement à un segment rectiligne respectif dans un plan contenant l'axe central.

11. Vis à empreinte hexalobulaire (51 ; 151), par exemple vis d'ostéosynthèse, la vis à empreinte hexalobulaire (51 ; 151) comprenant six lobes externes et six lobes internes répartis en alternance autour d'une direction axiale (X51 ; X151) de sorte que chaque lobe interne raccorde deux lobes externes respectifs,
la vis à empreinte hexalobulaire (51 ; 151) étant **caractérisée en ce que** :
i) les lobes externes comprennent :
- au moins trois lobes externes cylindriques (52 ; 152), chaque lobe externe cylindrique (52 ; 152) étant défini sensiblement par un cylindre respectif ayant un axe substantiellement parallèle à la direction axiale (X51 ; X151), et
- au moins deux lobes externes coniques (52.1 ; 152.1), chaque lobe externe conique (52.1 ; 152.1) étant défini sensiblement par un cône respectif ayant un axe substantiellement parallèle à la direction axiale (X51 ; X151), deux lobes externes coniques respectifs (52.1 ; 152.1) étant séparés par au moins un lobe externe cylindrique (52 ; 152),
et **en ce que** :
ii) les lobes internes comprennent :
- au moins trois lobes internes cylindriques (54 ; 154), chaque lobe interne cylindrique (54 ; 154) étant défini sensiblement par un cylindre respectif ayant un axe substantiellement parallèle à la direction axiale (X51 ; X151), et
- au moins deux lobes internes coniques (54.1 ; 154.1), chaque lobe interne conique (54.1 ; 154.1) étant défini sensiblement par un cône respectif ayant un axe substantiellement parallèle à la direction axiale (X51 ; X151), deux lobes internes coniques respectifs (54.1 ; 154.1) étant séparés par au moins un lobe interne cylindrique (54 ; 154).

12. Vis à empreinte hexalobulaire (51 ; 151) selon la revendication 11, dans lequel les lobes externes comprennent au moins trois lobes externes cylindriques (52 ; 152) et au moins deux lobes externes coniques (52.1 ; 152.1), et dans lequel les lobes internes sont constitués de six lobes internes cylindriques (54 ; 154).

13. Vis à empreinte hexalobulaire (51 ; 151) selon la revendication 11, dans lequel les lobes internes comprennent au moins trois lobes internes cylindriques (54 ; 154) et au moins deux lobes internes coniques (54.1 ; 154.1), et dans lequel les lobes externes sont constitués de six lobes externes cylindriques (52 ; 152).

## Patentansprüche

1. Anziehorgan (1; 101; 201; 301) zum Anziehen einer sechslappigen Schraube, beispielsweise einer Osteosyntheseschraube, wobei das Anziehorgan (1-301) sechs konvexe Lappen und sechs konkave Lappen umfasst, die abwechselnd um eine mittlere Achse (X1-X301) verteilt sind, sodass jeder konkave Lappen zwei aufeinanderfolgende konvexe Lappen um die mittlere Achse (X1-X301) miteinander verbindet,
wobei das Anziehorgan (1; 101; 201; 301) **dadurch gekennzeichnet ist, dass**:
i) die konvexen Lappen umfassen:
- mindestens drei zylindrische konvexe Lappen (2; 102; 202; 302), wobei jeder zylindrische konvexe Lappen (2-302) im Wesentlichen durch einen jeweiligen Zylinderabschnitt definiert ist, der eine Achse (X2-X302) aufweist, die im Wesentlichen parallel zur mittleren Achse (X1-X301) ist, und
- mindestens zwei konische konvexe Lappen (202.1; 302.1), wobei jeder konische konvexe Lappen (202.1; 302.1) im Wesentlichen durch einen jeweiligen Konus-Abschnitt definiert wird, der eine im Wesentlichen parallele Achse zur mittleren Achse (X1-X301) aufweist, wobei zwei jeweilige konische konvexe Lappen (202.1; 302.1) durch mindestens einen zylindrischen konvexen Lappen (202; 302) getrennt sind,
und/oder dadurch, dass:
ii) die konkaven Lappen umfassen:
- mindestens drei zylindrische konkave Lappen (4; 104; 204; 304), wobei jeder zylindrische konkave Lappen (4-304) im Wesentlichen durch einen jeweiligen Zylinderabschnitt definiert ist, der eine Achse (X4-X304) aufweist, die im Wesentlichen parallel zur mittleren Achse (X1-X301) ist, und
- mindestens zwei konische konkave Lappen (4.1; 104.1), wobei jeder konische konkave Lappen (4.1; 104.1) im Wesentlichen durch einen jeweiligen Konus-Abschnitt definiert wird, der eine Achse (X4.1; X104.1) aufweist, die im Wesentlichen parallel zur mittleren Achse (X1-X301) ist, wobei zwei jeweilige konische konkave Lappen (4.1; 104.1) durch mindestens einen zylindrischen konkaven Lappen (4-104) getrennt sind.

2. Anziehorgan (201; 301) nach Anspruch 1, wobei die konvexen Lappen mindestens drei zylindrische konvexe Lappen (202; 302) und mindestens zwei konische konvexe Lappen (202.1; 302.1) umfassen, und wobei die konkaven Lappen aus sechs zylindrischen konkaven Lappen (204; 304) gebildet werden.

3. Anziehorgan (1; 101) nach Anspruch 1, wobei die konkaven Lappen mindestens drei zylindrische konkave Lappen (4; 104) und mindestens zwei konische konkave Lappen (4.1; 104.1) umfassen, und wobei die konvexen Lappen aus sechs zylindrischen konvexen Lappen (2; 102) gebildet werden.

4. Anziehorgan (1/301) nach einem der vorstehenden Ansprüche, wobei die konkaven Lappen nur zwei konische konkave Lappen (4.1) umfassen, und/oder wobei die konvexen Lappen nur zwei konische konvexe Lappen (302.1) umfassen.

5. Anziehorgan (101/201) nach einem der vorstehenden Ansprüche, wobei die konkaven Lappen drei konische konkave Lappen (104.1) umfassen, und/oder wobei die konvexen Lappen drei konische konvexe Lappen (202.1) umfassen.

6. Anziehorgan (1-301) nach einem der vorstehenden Ansprüche, wobei jeder konische konvexe Lappen (202.1; 302.1) und/oder jeder konische konkave Lappen (4.1; 104.1) einen Halbwinkel (A) an der Spitze präsentiert, der zwischen 1 Grad und 5 Grad enthalten ist.

7. Anziehorgan (1) nach einem der vorstehenden Ansprüche, wobei jeder konische konkave Lappen (4.1) im Querschnitt in einer Ebene (P), die die mittlere Achse (X1) enthält, ein Profil präsentiert, das ein geradliniges Segment (4.2) umfasst, das sich von der freien Seite (1.0) des Anziehorgans (1) erstreckt und das auf die mittlere Achse (X1) geneigt ist, und/oder wobei jeder konische konvexe Lappen im Querschnitt in einer Ebene, die die mittlere Achse enthält, ein Profil präsentiert, das ein geradliniges Segment umfasst, das sich von der freien Seite des Anziehorgans erstreckt und das auf die mittlere Achse geneigt ist.

8. Anziehorgan (1) nach Anspruch 7, wobei jedes geradlinige Segment (4.2) eine Länge (L4.2) aufweist, die zwischen 0,3 mm und 4,0 mm enthalten ist.

9. Anziehorgan (1) nach einem der Ansprüche 7 bis 8, wobei sich jeder Schnittpunkt zwischen dem geradlinigen Segment (4.2) und der freien Seite (1.0) in einem Abstand (D4.2) befindet, der zwischen 30 % und 80 % des Abstands (D2) repräsentiert, der die mittlere Achse (X1) und die Spitze eines konvexen Lappens (2) trennt, wobei die Abstände senkrecht zur mittleren Achse (X1) gemessen werden.

10. Anziehorgan (1) nach einem der Ansprüche 7 bis 9, wobei das Profil jedes konischen konkaven Lappens (4.1) weiter ein krummliniges, beispielsweise kreisförmiges, Segment (4.4) umfasst, das sich tangential zu einem jeweiligen geradlinigen Segment (4.2) in einer Ebene (P) erstreckt, die die mittlere Achse (X1) enthält, wobei das krummlinige Segment (4.4) vorzugsweise einen Krümmungsradius (R4.4) aufweist, der zwischen 2 mm und 8 mm enthalten ist und eine Länge, die zwischen 0,5 mm und 2,0 mm enthalten ist, und/oder wobei das Profil jedes konischen konvexen Lappens weiter ein krummliniges Segment umfasst, das sich tangential zu einem jeweiligen geradlinigen Segment in einer Ebene erstreckt, die die mittlere Achse enthält.

11. Sechslappige Schraube (51; 151), beispielsweise eine Osteosyntheseschraube, wobei die sechslappige Schraube (51; 151) sechs äußere Lappen und sechs innere Lappen umfasst, die abwechselnd um eine axiale Richtung (X51; X151) verteilt sind, sodass jeder innere Lappen zwei jeweilige äußere Lappen miteinander verbindet,
wobei die sechslappige Schraube (51; 151) **dadurch gekennzeichnet ist, dass**:
i) die äußeren Lappen umfassen:
- mindestens drei zylindrische äußere Lappen (52; 152), wobei jeder zylindrische äußere Lappen (52; 152) im Wesentlichen durch einen jeweiligen Zylinder definiert ist, der eine Achse aufweist, die im Wesentlichen parallel zur axialen Richtung (X51; X151) ist, und
- mindestens zwei konische äußere Lappen (52.1; 152.1), wobei jeder konische äußere Lappen (52.1; 152.1) im Wesentlichen durch einen jeweiligen Konus definiert wird, der eine im Wesentlichen parallele Achse zur axialen Richtung (X51; X151) aufweist, wobei zwei jeweilige konische äußere Lappen (52.1; 152.1) durch mindestens einen zylindrischen äußeren Lappen (52; 152) getrennt sind,
und dadurch, dass:
ii) die inneren Lappen umfassen:
- mindestens drei zylindrische innere Lappen (54; 154), wobei jeder zylindrische innere Lappen (54; 154) im Wesentlichen durch einen jeweiligen Zylinder definiert ist, der eine Achse aufweist, die im Wesentlichen parallel zur axialen Richtung (X51; X151) ist, und
- mindestens zwei konische innere Lappen (54.1; 154.1), wobei jeder konische innere Lappen (54.1; 154.1) im Wesentlichen durch einen jeweiligen Konus definiert wird, der eine Achse aufweist, die im Wesentlichen zur axialen Richtung (X51; X151) parallel ist, wobei zwei jeweilige konische innere Lappen (54.1; 154.1) durch mindestens einen zylindrischen inneren Lappen (54; 154) getrennt sind.

12. Sechslappige Schraube (51; 151) nach Anspruch 11, wobei die äußeren Lappen mindestens drei zylindrische äußere Lappen (52; 152) und mindestens zwei konische äußere Lappen (52.1; 152.1) umfassen, und wobei die inneren Lappen aus sechs zylindrischen inneren Lappen (54; 154) gebildet werden.

13. Sechslappige Schraube (51; 151) nach Anspruch 11, wobei die inneren Lappen mindestens drei zylindrische innere Lappen (54; 154) und mindestens zwei konische innere Lappen (54.1; 154.1) umfassen, und wobei die äußeren Lappen aus sechs zylindrischen äußeren Lappen (52; 152) gebildet werden.

## Claims

1. A clamping member (1; 101; 201; 301), for clamping an hexalobular recess screw, for example an osteosynthesis screw, the clamping member (1-301) comprising six convex lobes and six concave lobes alternately distributed about a central axis (X1-X301) so that each concave lobe connects two consecutive convex lobes about the central axis (X1-X301),
the clamping member (1; 101; 201; 301) being **characterized in that**:
i) the convex lobes comprise:
- at least three cylindrical convex lobes (2; 102; 202; 302), each cylindrical convex lobe (2-302) being significantly defined by a respective cylinder portion having an axis (X2-X302) substantially parallel to the central axis (X1-X301), and
- at least two conical convex lobes (202.1; 302.1), each conical convex lobe (202.1; 302.1) being significantly defined by a respective cone portion having an axis substantially parallel to the central axis (X1-X301), two respective conical convex lobes (202.1; 302.1) being separated by at least one cylindrical convex lobe (202; 302),
and/or **in that**:
ii) the concave lobes comprise:
- at least three cylindrical concave lobes (4; 104; 204; 304), each cylindrical concave lobe (4-304) being significantly defined by a respective cylinder portion having an axis (X4-X304) substantially parallel to the central axis (X1-X301), and
- at least two conical concave lobes (4.1; 104.1), each conical concave lobe (4.1; 104.1) being significantly defined by a respective cone portion having an axis (X4.1; X104.1) substantially parallel to the central axis (X1-X301), two respective conical concave lobes (4.1; 104.1) being separated by at least one cylindrical concave lobe (4-104).

2. The clamping member (201; 301) according to claim 1, wherein the convex lobes comprise at least three cylindrical convex lobes (202; 302) and at least two conical convex lobes (202.1; 302.1), and wherein the concave lobes consist of six cylindrical concave lobes (204; 304).

3. The clamping member (1; 101) according to claim 1, wherein the concave lobes comprise at least three cylindrical concave lobes (4; 104) and at least two conical concave lobes (4.1; 104.1), and wherein the convex lobes consist of six cylindrical convex lobes (2; 102).

4. The clamping member (1/301) according to any one of the preceding claims, wherein the concave lobes comprise only two conical concave lobes (4.1), and/or wherein the convex lobes comprise only two conical convex lobes (302.1).

5. The clamping member (101/201) according to any one of the preceding claims, wherein the concave lobes comprise three conical concave lobes (104.1), and/or wherein the convex lobes comprise three conical convex lobes (202.1).

6. The clamping member (1-301) according to any one of the preceding claims, wherein each conical convex lobe (202.1; 302.1) and/or each conical concave lobe (4.1; 104.1) has a half-angle (A) at the top comprised between 1 degree and 5 degrees.

7. The clamping member (1) according to any one of the preceding claims, wherein each conical concave lobe (4.1) has, in section in a plane (P) containing the central axis (X1), a profile comprising a rectilinear segment (4.2) which extends from the free face (1.0) of the clamping member (1) and which is titled on the central axis (X1), and/or in which each conical convex lobe has, in section in a plane containing the central axis, a profile comprising a rectilinear segment which extends from the free face of the clamping member and which is titled on the central axis.

8. The clamping member (1) according to claim 7, wherein each rectilinear segment (4.2) has a length (L4.2) comprised between 0.3 mm and 4.0 mm.

9. The clamping member (1) according to any one of claims 7 to 8, each intersection between each rectilinear segment (4.2) and the free face (1.0) is located at a distance (D4.2) representing between 30% and 80% of the distance (D2) separating the central axis (X1) and the top of a convex lobe (2), the distances being measured perpendicularly to the central axis (X1).

10. The clamping member (1) according to any one of claims 7 to 9, wherein said profile of each conical concave lobe (4.1) further comprises a curvilinear segment (4.4), for example circular segment, tangentially extending to a respective rectilinear segment (4.2) in a plane (P) containing the central axis (X1), the curvilinear segment (4.4) preferably having a radius of curvature (R4.4) comprised between 2 mm and 8 mm and a length comprised between 0.5 mm and 2.0 mm, and/or wherein said profile of each conical convex lobe further comprises a curvilinear segment tangentially extending to a respective rectilinear segment in a plane containing the central axis.

11. An hexalobular recess screw (51; 151), for example an osteosynthesis screw, the hexalobular recess screw (51; 151) comprising six outer lobes and six inner lobes alternately distributed about an axial direction (X51; X151) so that each inner lobe connects two respective outer lobes,
the hexalobular recess screw (51; 151) being **characterized in that**:
i) the outer lobes comprise:
- at least three cylindrical outer lobes (52; 152), each cylindrical outer lobe (52; 152) being significantly defined by a respective cylinder having an axis substantially parallel to the axial direction (X51; X151), and
- at least two conical outer lobes (52.1; 152.1), each conical outer lobe (52.1; 152.1) being significantly defined by a respective cone having an axis substantially parallel to the axial direction (X51; X151), two respective conical outer lobes (52.1; 152.1) being separated by at least one cylindrical outer lobe (52; 152), and/or **in that**:
ii) the inner lobes comprise:
- at least three cylindrical inner lobes (54; 154), each cylindrical inner lobe (54; 154) being significantly defined by a respective cylinder having an axis substantially parallel to the axial direction (X51; X151), and
- at least two conical inner lobes (54.1; 154.1), each conical inner lobe (54.1; 154.1) being significantly defined by a respective cone having an axis substantially parallel to the axial direction (X51; X151), two respective conical inner lobes (54.1; 154.1) being separated by at least one cylindrical inner lobe (54; 154).

12. The hexalobular recess screw (51; 151) according to claim 11, wherein the outer lobes comprise at least three cylindrical outer lobes (52; 152) and at least two conical outer lobes (52.1; 152.1), and wherein the inner lobes consist of six cylindrical inner lobes (54; 154).

13. The hexalobular recess screw (51; 151) according to claim 11, wherein the inner lobes comprise at least three cylindrical inner lobes (54; 154) and at least two conical inner lobes (54.1; 154.1), and wherein the outer lobes consist of six cylindrical outer lobes (52; 152).
